# EUROPEAN PATENT SPECIFICATION

(11) **EP 4 340 628 B1**
(45) Date of publication and mention of the grant of the patent: **10.09.2025**
(21) Application number: 22747110.9
(22) Date of filing: 17.05.2022
(51) Int. Cl.: A23G 3/00, A23G 3/34, A23G 3/36, A23L 27/00, A61K 9/50

(54) **FLAVOR BEADS AND METHOD OF MAKING AND USING SAME**
GESCHMACKSKÜGELCHEN SOWIE VERFAHREN ZU IHRER HERSTELLUNG UND VERWENDUNG
BILLES D'ARÔME ET LEUR PROCÉDÉ DE FABRICATION ET D'UTILISATION

(30) Priority: 20.05.2021 US 202163191043 P
(43) Date of publication of application: 27.03.2024
(73) Proprietor: V. Mane Fils, 06620 Le Bar-sur-Loup (FR)
(72) Inventor: COLE, Jason C., Georgetown, Ohio 45121 (US); WIELAND, Robert B., Cincinnati, Ohio 45248 (US)
(74) Representative: Murgitroyd & Company
(86) International application number: PCT/IB2022/000273
(87) International publication number: WO 2022/243747

(56) References cited:
- EP-A1- 2 540 287
- WO-A1-2020/115139
- JP-A- 2011 084 482

## Description

### FIELD OF THE INVENTION

The present invention relates generally to encapsulated flavor compositions and methods of manufacturing and using the encapsulated flavor compositions.

### BACKGROUND OF THE INVENTION

Various types of chewable articles are known in commerce. These articles include foodstuff, such as confectionery items. The chewable articles often include various types of active agents or ingredients. Non-limiting examples of typical active ingredients include flavors, sweeteners, colors, medicaments, vitamins, minerals, and sensates.

Frequent problems associated with the application of flavor systems in chewable articles are the loss of flavor by volatilization and short-lasting sensory performance. A common approach to address these problems is the use of encapsulation. Encapsulation is broadly defined as a technology of packaging solids, liquids, or gaseous materials in small, sealed matrices or capsules that can release their contents at controlled rates under specific conditions. In addition to the foregoing, active ingredients may be encapsulated for a variety of other reasons, such as enhanced retention, protection from undesirable interactions with the bulk matrix or other ingredients, guard against either light-induced reactions or oxidation, and/or to effect the controlled release of the ingredient.

However, the final physical or mechanical properties of the capsules also need to match the requirements of their intended application, such as an ability to withstand processing forces (e.g., shear and/or compression) encountered during incorporation into consumer products, such as stick gum or compressed tablets. In addition, to provide consistent and/or enhanced flavor sensory experience (e.g., sufficient intensity and/or long lasting effect) to chewable articles, it is desirable that the encapsulated materials contain a sufficient quantity of the active ingredient(s) and are homogenously dispersed. Thus, depending on the desired flavor loading, particle size, solubility characteristic, and texture properties, there are several different types of encapsulation techniques to choose from, such as *in-situ* gelation, coacervation, extrusion, coextrusion, spray dry, or spray dry granulation.

Expired U.S. Patent No. 6,045,835 to Soper et al. describes a method of encapsulating flavors by controlled water transport across a hydrophilic hydrocolloid shell of a microcapsule into a blank oily core. The microcapsules were prepared by complex coacervation, which generally yields microcapsules typically 100 to 400 microns in diameter having large particle size distributions. However, complex coacervation particles typically possess thin shell walls, relative to their core, which result in weak texture properties.

Expired U.S. Patent No. 6,436,461 to Bouwmeesters et al. describes using acid polysaccharide (e.g., alginate) beads as food additives, where its matrix contains active ingredients like flavors. Initially, blank (unflavored oily phase) gel beads are made from an emulsion and then dried. The dried "blank" gel beads are loaded with flavor by mixing the beads with the flavor, which is slowly absorbed or adsorbed into the oily phase portion of the beads. The flavor-loaded alginate beads are reported to span average particle size diameters from 10 to 5000 microns, and various bead sizes were separated using sieves. The larger bead particles (1 to 2 mm) demonstrated higher aroma intensity versus the smaller sizes (i.e., 0.5 to 1 mm; 0.25 to 0.5 mm; 0 to 0.25 mm; and unencapsulated flavors). However, the maximum flavor loading in the gel beads was only about 20 wt%.

Expired U.S. Patent No. 6,325,859 to De Roos et al. describes beads consisting of a matrix of a reticulated, multivalent cation containing acid polysaccharide and at least one oil insoluble liquid active ingredient and/or one oil insoluble solid active ingredient filling at least partly the voids built by the acid polysaccharide. The active ingredient advantageously is at least one compound of the group consisting of flavors, fragrances, vitamins or coloring materials. The process forms a system of a solution, dispersion, or emulsion of either a liquid or solid active ingredient in an aqueous solution of an acid polysaccharide, especially in the form of an alkali metal salt, an emulsifier, and optionally one or more other water soluble or water dispersible substances. Discrete droplets of the system are then formed. The droplets are converted to water-insoluble gel beads by introducing the droplets in an aqueous or alcoholic solution containing multivalent cations (e.g., calcium), thereby building a suspension of beads. The flavor-loaded gel beads are reported to span average particle size diameters from 10 to 5000 microns, and the flavor loading may range from 0.1 wt% to more than 80 wt%, flavors containing water-soluble constituents are prone to leaching into the cross-linking bath.

Expired U.S. Patent No. 4,689,235 to Barnes et al. describes an extrudable encapsulation system for oils, flavors, etc. comprising maltodextrin and CAPSUL^{®}. While these extruded particles are reportedly capable of containing up to 40 wt% flavor loadings, the flavor loaded extruded particles require milling and sieving to obtain encapsulated flavor particles with the desired particle size and distribution.

Expired U.S. Patent No. 2,886,446 to Kramer et al. discloses encapsulating or dispersing a water-immiscible flavoring agent within finely divided particles of gelatin. Typically, the process will be carried out by emulsifying and dispersing the volatile water-immiscible flavoring oil in the form of discrete or minute micro-droplets throughout an aqueous solution of gelatin, and drying the emulsion so formed. Exemplified methods include tunnel or slab drying, foam drying, and spray drying. Slab drying and foam drying required grinding of the dried product, where the former was reported to be ground into particles between 20 mesh and 35 mesh (840-500 microns), and the latter reported to be ground into particles between 20 mesh and 400 mesh (840-37 microns). Without sieving, milled samples typically have large particle size distributions. Reported particles sizes in the range of 40-80 microns were obtained from spray drying.

Expired U.S. Patent No. 4,386,106 to Merritt et al. describes a delayed release encapsulated flavorant composition prepared from an aqueous emulsion of a flavorant in a hydrophilic encapsulation material comprising gelatin, a natural gum, and plasticizer. The solid dried emulsion (e.g., sheet) is milled to a mesh size of between 20-30 (840 - 595 microns) to produce solid powder, which is then coated with a water insoluble material to produce an encapsulated flavorant having a relatively thin water-insoluble coating. Without sieving, milled samples typically have large particle size distributions.

U.S. Patent No. 6,974,592 to Yan describes a microcapsule comprising an agglomeration of primary microcapsules based on complex coacervation, each individual primary microcapsule having a primary shell and the agglomeration being encapsulated by an outer shell. The primary microcapsules (primary shells) typically have an average diameter of about 40 nm to about 10 µm, and the encapsulated agglomerations (outer shells) may have an average diameter of from about 1 µm to about 2000 µm. Without sieving, agglomerated samples typically have large particle size distributions. WO2020115139 A1 discloses a method for enhancing a flavor sensory experience in a confectionary product. JP2011084482 A discloses a multi-core microcapsule. EP2540287 A1 discloses a continuous process for the preparation of gelatin based nanoparticles.

Accordingly, there is a need for new encapsulated flavors having high flavor loadings with desirable rigidity properties, good dispersibility, and monodispersity, which can provide long lasting flavor.

### SUMMARY OF THE INVENTION

The present invention is defined by the appended claims. There are additional features and advantages of the subject matter described herein. They will become apparent as this specification proceeds. The various features described in the claims and below for various embodiments may be used in combination or separately. For example, specified ranges may be inclusive of their recited endpoints, unless explicitly excluded. Any particular embodiment need not provide all features noted above, nor solve all problems or address all issues noted above.

In accordance with an embodiment of the present invention, an encapsulated flavor delivery system is provided, comprising a plurality of dried spherical beads, each in the form of a continuous gelled matrix comprising an uncross-linked gelatin and a filler, said matrix surrounding a plurality of droplets comprising an oily liquid flavor composition containing a flavorant. The continuous gelled matrix is substantially void of acid polysaccharide gelling agents, said continuous gelled matrix comprising less than 1 wt% of acid polysaccharide gelling agents, based on the entire weight of the continuous gelled matrix. The plurality of flavor droplets has an average diameter in a range from about 1 micron to about 20 microns; and the beads are spherical having an average particle diameter in a range from about 400 microns to about 2000 microns, with a coefficient of variance less than 15%.

In accordance with another embodiment of the present invention, a method for making an encapsulated flavor delivery system is provided. The method comprises: forming an emulsion composition comprising a plurality of droplets of an oily liquid flavor composition comprising a flavorant within a gellable mixture comprising an aqueous solution of gelatin and a filler, wherein the plurality of flavor droplets have an average diameter in a range from about 1 micron to about 20 microns, wherein the gellable mixture is substantially void of acid polysaccharide gelling agents such that the continuous gelled matrix comprises less than 1 wt% of acid polysaccharide gelling agents, based on the entire weight of the continuous gelled matrix; extruding the emulsion composition through a nozzle immersed in a non-aqueous fluid at a temperature at least 10°C below a gelling temperature of the gellable mixture to form wet spherical beads comprising a continuous gelled matrix comprising uncross-linked gelatin and the filler, said matrix surrounding the plurality of droplets of the oily liquid flavor composition; isolating the wet spherical beads from the non-aqueous fluid; and drying the wet spherical beads to form the encapsulated flavor delivery system comprising dried spherical beads having an average particle diameter in a range from about 400 microns to about 2000 microns, with a coefficient of variance less than 15%.

The encapsulated flavor delivery system may be used in a variety of applications, such as chewing gum, tablet, taffy, and other chewable confectionary products.

Another embodiment of the invention relates to a confectionary product comprising the encapsulated flavor delivery system according to the invention.

Another embodiment of the invention relates to the use of the encapsulated flavor delivery system according to the invention, or susceptible of being obtained by the method according to the invention, for providing a sustained release of the original flavor notes of a confectionary product comprising the encapsulated flavor delivery system.

### BRIEF DESCRIPTION OF THE DRAWINGS

The accompanying drawing, which are incorporated in and constitute a part of this specification, illustrate embodiments of the invention and, together with a general description of the invention given above, and the detailed description given below, serve to explain the invention. It will be appreciated that for purposes of clarity and where deemed appropriate, reference numerals have been repeated in the figures to indicate corresponding features.
FIG. 1 is a Scanning Electron Micrograph (SEM) picture of a dried spherical bead, in accordance with an embodiment of the present invention;
FIG. 2 is an SEM picture showing of an internal portion or cross-section of a dried spherical bead, in accordance with an embodiment of the present invention;
FIG. 3 is a chart showing a sensory comparison of flavor perception between the encapsulated flavor delivery system in accordance with an embodiment of the present invention versus prior art flavor delivery systems in a chewing gum confectionary application; and
FIG. 4 is a chart showing a sensory comparison of menthol bitterness perception for the encapsulated flavor delivery system void of any sweetener, as well as containing a sweetener i) in the continuous gelled matrix, ii) in the oily liquid flavor composition, and iii) in each of the continuous gelled matrix and the oily liquid flavor composition, in a chewing gum confectionary application.

### DETAILED DESCRIPTION

Unless otherwise explained, all technical and scientific terms used herein have the same meaning as commonly understood by one of ordinary skill in the art to which this disclosure belongs. In case of conflict, the present specification, including explanations of terms, will control. The singular terms "a," "an," "the", and "at least one" include plural referents unless context clearly indicates otherwise. Similarly, the word "or" is intended to include "and" unless the context clearly indicates otherwise. The term "comprising" means "including" or "containing"; hence, "comprising A or B" means including A or B, as well as A and B together.

In accordance with an embodiment of the present invention, an encapsulated flavor delivery system is provided, comprising: a plurality of dried spherical beads, each in the form of a continuous gelled matrix comprising an uncross-linked gelatin and a filler, said matrix surrounding a plurality of droplets comprising an oily liquid flavor composition containing a flavorant. The continuous gelled matrix is substantially void of acid polysaccharide gelling agents. The plurality of flavor droplets has an average diameter in a range from about 1 micron to about 20 microns; and the beads are spherical having an average particle diameter in a range from about 400 microns to about 2000 microns, with a coefficient of variance less than 15%.

As used herein, the "coefficient of variance" is a measure of relative variability and is expressed as a percentage by dividing the standard deviation by the mean and then multiplying by 100.

As used herein the "gellable mixture" comprises (consists essentially of, or consists of) an aqueous solution comprising a gelatin and a filler, optionally with one or more other gelling agents, fillers, and/or additives, that is able to convert an aqueous flowable liquid to a solid or a gel upon cooling to a temperature below a gelling point of the gellable mixture.

As used herein, "uncross-linked gelatin" means the absence of ionic or covalent linkages, arising from treatment with chemical or enzymatic cross-linking agents, between one section of the gelatin gelling agent to another section in a gelled matrix.

As used herein, "spherical" includes a distorted sphere where its shape ratio, which is a ratio of width/length measured by microscopy (SZX9 Olympus microscope with MICROVISION software), is at least 0.8 or greater, such as 0.9 or greater.

As used herein, "substantially void of acid polysaccharide gelling agents" means that the continuous gelled matrix comprises less than 1 wt% of acid polysaccharide gelling agents, based on the entire weight of the continuous gelled matrix. For example, in an embodiment, the content of any acid polysaccharide gelling agent is less than 0.1 wt%. In another embodiment, no acid polysaccharide gelling agents are intentionally added to the gellable mixture. In an embodiment, the continuous gelled matrix is void of polysaccharide gelling agents.

### GELLABLE MIXTURE

Embodiments of the present invention are premised on the realization that uncross-linked gelatin beads, characterized by a defined size and derived from an emulsion of gelatin, filler, and oily liquid flavor, provide a slow release of active ingredients, e.g., flavorant, in chewable confectionary applications. In accordance with embodiments of the present invention, the gellable mixture comprises a sufficient quantity of a gelatin and filler, which upon cooling the gellable mixture to a temperature below a gelling temperature of the gellable mixture, forms a gelled matrix. Non-limiting examples of gelatin sources include, but are not limited to bovine, porcine, fish, non-animal based gelatin obtained by fermentation, such as Geltor^{®} (by Geltor, Inc. of San Leandro, CA, USA), and combinations thereof.

The strength of a gelatin gel can be determined by a Bloom gelometer, and is indicated by a Bloom number. The test was originally developed in 1925 by O. T. Bloom (U.S. Pat. Nos. 1,540,979 and 2,119,699). The test determines the weight (in grams) needed by a probe (normally with a diameter of 0.5 inch) to deflect the surface of the gel 4 mm without breaking it. The result is expressed in Bloom (grades), which is usually between 30 and 325 Bloom. The higher the Bloom number, the stronger the gel. To perform the Bloom test on gelatin, a 6.67% gelatin solution is made at 60° C., then kept for 17-18 hours at 10° C prior to being tested.

The suitable range for Bloom values of the gelatin employed may vary widely. Suitable gelatins include those having a Bloom value between 150 and 300. For example, the Bloom value may be 150, 175, 200, 225, 250, 275, 300, or in a range between any two of the foregoing. In an embodiment, the gelatin has a bloom value between 150 to 300, or 175 to 300, or 200 to 300, or 250 to 300. In a preferred embodiment, the Bloom value of the gelatin is in a range from 250 to 300.

The gelatin content in the gellable mixture may be in a range of 5 weight percent to 95 weight percent, wherein weight percentage is based on the total mass of the dry weight (non-water) components of the gellable mixture. The gelatin may be present in the gellable mixture in an amount of 5.0 wt%, 7.5 wt%, 10 wt%, 12.5 wt%, 15 wt%, 17.5 wt%, 20 wt%, 25 wt%, 35 wt%, 45 wt%, 50 wt%, 60 wt%, 70 wt%, 80 wt%, 90 wt%, 95 wt%, or in a range between any two of the foregoing. For example, in an embodiment, the gelatin content of the gellable mixture is between 5 wt% to 95 wt%, or 15 wt% to 95 wt%, or 50 wt% to 95 wt%.

In accordance with embodiments of the present invention, the gellable mixture further comprises a filler, which may be a material that can increase the percentage of dry material (non-water components) in the gellable mixture and thus in the obtained gelled matrix after extrusion and cooling. Increasing the dry material amount in the gellable mixture assists in solidifying the gelled matrix, and may improve drying of the concomitant hydrated (wet) gelatin bead. In an aspect, the filler may further act as an antiplasticizer making the gelled matrix physically more resistant to deformation or breakage. In another aspect, the filler may further act as a plasticizer, which improves the processability of the gellable mixture and/or the flexibility of the gelled matrix. Exemplary fillers may include, but are not limited to starch derivatives such as dextrin, maltodextrin, innulin, sucrose, allulose, tagatose, cyclodextrin (alpha, beta, gamma, or modified cyclodexrin); cellulose derivatives such as microcrystalline cellulose (MCC) hydroxypropylmethylcellulose (HPMC), hydroxypropylcellulose (HPC), methylcellulose (MC), or carboxymethylcellulose (CMC); a polyvinyl alcohol; polyols with non-plasticizing properties, such as pre-gelatinized starches; polyols with plasticizing properties, such as trehalose, erythritol, sorbitol, maltitol, mannitol, xylitol, propylene glycol, glycerol, triacetine, or a polyethylene glycol; or combinations of two or more of the foregoing. Preferably, the filler is selected from polyols, such as trehalose, erythritol, sorbitol, maltitol, mannitol, xylitol, propylene glycol, glycerol, triacetine, or a polyethylene glycol; or combinations of two or more of the foregoing. More preferably, the filler comprises (consist essentially of, or consist of) sorbitol.

Based on a total mass of the dry weight ingredients, the filler may be present in the gellable mixture in an amount in the range from about 0.1 to about 60 wt%. For example, the filler may be present in the gellable mixture in an amount of 0.1 wt%, 0.2 wt% 0.5 wt%, 0.8 wt%, 1.0 wt%, 1.5 wt% 2.0 wt%, 2.5 wt%, 3.0 wt%, 4.0 wt%, 5.0 wt%, 7.5 wt%, 8 wt%, 10 wt%, 12.5 wt%, 15 wt%, 17.5 wt%, 20 wt%, 25 wt%, 35 wt%, 45 wt%, 50 wt%, 60 wt%, or in a range between any two of the foregoing. In an embodiment, the filler is present in the gellable mixture in an amount within a range from 0.1 wt% to 60 wt%, or 8 wt% to 50 wt%, or 10 wt% to 25 wt%.

In an embodiment, the filler is selected from sorbitol, glycerol, mannitol, sucrose, trehalose, propylene glycol, xylitol, erythritol, or combinations thereof, and is present in the gellable mixture in a range from 7.5 wt% to 45 wt%, based on a total mass of the dry weight ingredients.

In accordance with embodiments of the present invention, the gellable mixture may further comprise sweeteners. The sweetener may be a hydrophilic sweetener homogenously dispersed throughout the continuous gelled matrix. Exemplary hydrophilic sweeteners include, but are not limited to, monosaccharides, disaccharides, sugar alcohols, aspartame, acesulfame potassium, saccharin (optionally as sodium, potassium, or calcium salt), cyclamate (optionally as sodium or calcium salt), or combinations thereof. In an embodiment, the hydrophilic sweetener comprises acesulfame potassium, present in the gellable mixture in a sufficient quantity to provide about 0.5 to 3 wt%, preferably about 1 wt%, of acesulfame potassium in the dried gelatin bead.

In accordance with an embodiment of the present invention, the gellable mixture may further comprise one or more additives, such as colorants, opacifiers, humectants, preservatives, flavorings, and buffering salts and acids. Opacifiers may be used to opacify the gelled matrix when the encapsulated active agents are light sensitive. Exemplary opacifiers include titanium dioxide, zinc oxide, calcium carbonate and combinations thereof. Colorants can be used to provide color to the gelled matrix and/or for product identification/differentiation purposes. Suitable colorants include synthetic and natural dyes and combinations thereof. Accordingly, the gellable mixture may further include the synthetic or natural coloring agents that is water soluble or capable of forming a water-stable suspension. Exemplary coloring agents include, but are not limited to, pigments, titanium dioxide, iron oxides, carbon black, or any type of food, oral care, cosmetic or pharmaceutical pigments, such as colors distributed by Sensient Colors (St. Louis, MO). Natural coloring agents may also be obtained from Kancor Ingredients, Ltd (Kerala, India), including the natural pigments sold under Kancor's C-CAPTURE's color stabilization process. Additionally, the gellable mixture may further include other additives, such as actives, sensates, and pH modifiers.

Humectants can be used to suppress the water activity of the gelled matrix. Suitable humectants include glycerin and sorbitol, which are often components of the filler composition. Due to the low water activity of dried, properly stored beads, the greatest risk from microorganisms comes from molds and yeasts. For this reason, preservatives can be incorporated into the gellable mixture. Suitable preservatives include alkyl esters of p-hydroxy benzoic acid such as methyl, ethyl, propyl, butyl and heptyl (collectively known as "parabens") or combinations thereof.

In accordance with embodiments of the present invention, the gellable mixture is an aqueous mixture of the gelatin, filler, etc. in water. A typical weight ratio of water to the non-water (dry) ingredients is in a range from 1:1 to 20:1. Preferably, the water used for the gellable mixture is purified water, such as distilled water, deionized water, or reverse osmosis water, but processing water is viable. The ingredients are combined to form the gellable mixture. To minimize foaming or incorporation of air, the dry weight ingredients are gently mixed with water heated to a temperature above 50°C, such as 65°C. The prepared gellable mixture is transferred to preheated, temperature-controlled, jacketed holding tanks where the gellable mixture is aged at 50°C to 80°C until mixed with the oily liquid flavor.

The gellable mixture can also further include preservatives or bactericides such as benzoate, parabens, diols, cetylpyridinium chloride, diazolidinyl urea or any preservatives used for food, pharmaceutical or cosmetic products. Such preservatives may be useful if the product spherical beads are not sufficiently dried to inhibit growth of bacteria, molds, and yeasts (i.e., a water activity (Aw) equal to 0.6 or less). Water activity (Aw), as known by one skilled in the art, is sometimes referred to as "free" or "available" water in a system that is not bound to non-aqueous constituents. It can properly be defined as the partial vapor pressure of food moisture divided by the equilibrium vapor pressure of pure water at the same temperature. Water activity value can be measured using a LabMaster-aw by Novasina AG (Lachen, Switzerland), at 25°C.

The gelling temperature of the gellable mixture may advantageously be determined by analyzing the rheological profile as a function of temperature using a rheometer (Haake- Mars III) with a cone of 35mm/2°, by oscillation, strain= 0.001 (0.1%), frequency = 1Hz, and decreasing temperature of the sample from 90°C to 10°C (3°C/min). A plot of the viscoelastic moduli (Pascal) vs temperature (°C) will indicate the gelling temperature (°C) as the crossover of moduli (G'=G").

### OILY LIQUID FLAVOR COMPOSITION

In accordance with embodiments of the present invention, the oily liquid flavor composition comprises a flavorant, and may also include one or more hydrophobic oils or solvents conventionally used in the food, pharmaceutical, or cosmetic industries. As used herein, the term "flavorant" may be used interchangeably with "flavoring", or "flavor substance".

The hydrophobic oils may include triglycerides, and in particular medium chain triglycerides (MCT), such as triglycerides of caprylic or capric acids, borage oil, vegetable oil, olive oil, sunflower oil, corn oil, pecan nut oil, pistachio kernel oil, rapeseed oil, rice germ oil, sesame seed oil, Soya oil, groundnut oil, hazelnut oil, walnut oil, coconut oil, pumpkin seed oil, linseed oil, maize germ oil, macadamia nut oil, almond oil, grapeseed oil, wheatgerm oil, thistle oil, castor oil, mineral oils, silicone oils; or fractionated coconut oils, which mainly have fatty acid residues with a length of between six and eight carbon atoms (C6- to C8-fatty acids). Diluent solvents may also be used, such as propylene glycol, diacetine (glycerine diacetate), triacetine (glycerine triacetate), benzyl alcohol, triethyl citrate, ethyl lactate, isopropanol, ethanol, glycerine, or combinations thereof.

The oily liquid flavor composition may include one or more low melting substances, such as low melting waxes, fatty acids, triglycerides, polyglycerol esters, or the like. Non-limiting examples of low melting substances include cocoa butter oil, coprah oil, bees waxes, castor oil, butter fat, or the like. In an embodiment, the oily liquid flavor comprises a medium chain triglyceride having a melting point of about 30°C or less.

The flavorant(s) may be mixed with one or more of the abovementioned oils or solvents and then used in accordance with the embodiments described herein. Preferably the flavorant(s) used according to the invention comprises lipophilic flavor substances. Lipophilic flavor substances are preferably used in the context of the present invention and thus preferably used in the oily liquid flavor. They belong to various chemical groups, such as the group comprising hydrocarbons, aliphatic alcohols, aliphatic aldehydes and the acetals thereof, aliphatic ketones and oximes thereof, aliphatic sulfur-containing compounds, aliphatic nitriles, aliphatic carboxylic acids esters, acyclic terpene alcohols, acyclic terpene aldehydes and ketones, cyclic terpene alcohols, cyclic terpene aldehydes and ketones, cyclic alcohols, cycloaliphatic carboxylic acids, aromatic hydrocarbons, araliphatic alcohols, esters of araliphatic alcohols and aliphatic carboxylic acids, araliphatic ethers, aromatic and araliphatic aldehydes, aromatic and araliphatic ketones, aromatic and araliphatic carboxylic acids and the esters, nitrogenous aromatic compounds, phenols, phenyl ethers, phenyl esters heterocyclic compounds, lactones, and combinations thereof.

The lipophilic flavor substances particularly preferably used in the context of the present invention have a log P_{O/W} of higher than 1.0 and are preferably selected from the group consisting of: acetophenone, allyl capronate, alpha-ionone, beta-ionone, anisaldehyde, anisyl acetate, anisyl formate, benzaldehyde, benzothiazole, benzyl acetate, benzyl alcohol, benzyl benzoate, beta-ionone, butyl butyrate, butyl caproate, butylidene phthalide, carvone, camphene, caryophyllene, cineol, cinnamyl acetate, citral, citronellol, citronellal, citronellyl acetate, cyclohexyl acetate, cymol, damascone, decalactone, dihydrocoumarin, dimethyl anthranilate, dimethyl anthranilate, dodecalactone, ethoxyethyl acetate, ethylbutyric acid, ethyl butyrate, ethyl caprinate, ethyl capronate, ethyl crotonate, ethyl furaneol, ethyl guajacol, ethyl isobutyrate, ethyl isovalerate, ethyl lactate, ethyl methyl butyrate, ethyl propionate, eucalyptol, eugenol, ethyl heptylate, 4-(p-hydroxyphenyl)-2-butanone, gamma-decalactone, geraniol, geranyl acetate, geranyl acetate, grapefruit aldehyde, methyl dihydrojasmonate (e.g. hedione), heliotropin, 2-heptanone, 3-heptanone, 4-heptanone, trans-2-heptenal, cis-4-heptenal, trans-2-hexenal, cis-3-hexenol, trans-2-hexenoic acid, trans-3-hexenoic acid, cis-2-hexenyl acetate, cis-3-hexenyl acetate, cis-3-hexenyl capronate, trans-2-hexenyl capronate, cis-3-hexenyl formate, cis-2-hexyl acetate, cis-3-hexyl acetate, trans-2-hexyl acetate, cis-3-hexyl formate, para-hydroxy benzyl acetone, isoamyl alcohol, isoamyl isovalerate, isobutyl butyrate, isobutyraldehyde, isoeugenol methyl ether, isopropylmethylthiazole, lauric acid, levulinic acid, linalool, linalool oxide, linalyl acetate, menthol, menthofuran, methyl anthranilate, methylbutanol, methylbutyric acid, 2-methylbutyl acetate, methyl capronate, methyl cinnamate, 5-methyl furfural, 3,2,2-methyl cyclopentenolone, 6,5,2-methyl heptenone, methyl dihydrojasmonate, methyl jasmonate, 2-methyl methyl butyrate, 2-methyl-2-pentenoic acid, methylthiobutyrate, 3,1-methylthiohexanol, 3-methylthiohexyl acetate, nerol, neryl acetate, trans,trans,2,4-nonadienal, 2,4-nonadienol, 2,6-nonadienol, 2,4-nonadienol, nootkatone, delta-octalactone, gamma-octalactone, 2-octanol, 3-octanol, 1,3-octenol, 1-octyl acetate, 3-octyl acetate, palmitic acid, paraldehyde, phellandrene, pentanedione, phenylethyl acetate, phenylethyl alcohol, phenylethyl alcohol, phenylethyl isovalerate, piperonal, propionaldehyde, propyl butyrate, pulegone, pulegol, sinensal, sulfurol, terpinene, terpineol, terpinolene, 8,3-thiomenthanone, 4,4,2-thiomethyl pentanone, thymol, delta-undecalactone, gamma-undecalactone, valencene, valeric acid, vanillin, acetoin, ethyl vanillin, ethyl vanillin isobutyrate, 2,5-dimethyl-4-hydroxy-3(2H)-furanone, homofuraneol, homofuronol, 5-ethyl-2-methyl-4-hydroxy-3(2H)-furanone, maltol and maltol derivatives, coumarin and coumarin derivatives, gamma-lactones, gamma-undecalactone, gamma-nonalactone, gamma-decalactone, delta-lactones, 4-methyl delta decalactone, massoia lactone, delta decalactone, tuberose lactone, methyl sorbate, divanillin, 4-hydroxy-2(or 5)-ethyl-5(or 2)-methyl-3(2H)furanone, 2-hydroxy-3-methyl-2-cyclopentenone, 3-hydroxy-4,5-dimethyl-2(5H)-furanone, acetic acid isoamyl ester, butyric acid ethyl ester, butyric acid-n-butyl ester, butyric acid isoamyl ester, 3-methylbutyric acid ethyl ester, n-hexanoic acid ethyl ester, n-hexanoic acid allyl ester, n-hexanoic acid-n-butyl ester, n-octanoic acid ethyl ester, ethyl-3-methyl-3-phenyl glycidate, ethyl-2-trans-4-cis-decadienoate, 4-(p-hydroxyphenyl)-2-butanone, 1,1-dimethoxy-2,2,5-trimethyl-4-hexane, 2,6-dimethyl-5-hepten-1-al and phenyl-acetaldehyde, 2-methyl-3-(methylthio)furan, 2-methyl-3-furanthiol, bis(2-methyl-3-furyl)disulfide, furfuryl mercaptan, methional, 2-acetyl-2-thiazoline, 3-mercapto-2-pentanone, 2,5-dimethyl-3-furanthiol, 2,4,5-trimethylthiazole, 2-acetylthiazole, 2,4-dimethyl-5-ethylthiazole, mercapto-3-methyl-1-butanol, 2-acetyl-1-pyrroline, 2-methyl-3-ethylpyrazine, 2-ethyl-3,5-dimethylpyrazine, 2-ethyl-3,6-dimethylpyrazine, 2,3-diethyl-5-methylpyrazine, 3-isopropyl-2-methoxypyrazine, 3-isobutyl-2-methoxypyrazine, 2-acetylpyrazine, 2-pentylpyridine, (E,E)-2,4-decadienal, (E,E)-2,4-nonadienal, (E)-2-octenal, (E)-2-nonenal, 2-undecenal, 12-methyltridecanal, 1-penten-3-one, 4-hydroxy-2,5-dimethyl-3(2H)-furanone, guajacol, 3-hydroxy-4,5-dimethyl-2(5H)-furanone, 3-hydroxy-4-methyl-5-ethyl-2(5H)-furanone, cinnamaldehyde, cinnamyl alcohol, methyl salicylate, isopulegol and further stereoisomers, enantiomers, positional isomers, diastereomers, cis/trans-isomers or epimers (not expressly mentioned) of these substances.

The oily liquid flavor composition may include natural or synthetic aromas and/or fragrances, as well as natural oils or extracts. Non-limiting examples of suitable aromas are vanilla, coffee, chocolate, cinnamon, or mint. Non-limiting examples of suitable fragrances are fruity, confectionery, floral, sweet, or woody fragrances. Non-limiting examples of suitable natural oils or extracts include peppermint oils, spearmint oils, eucalyptus oils, wintergreen oils, cinnamon oils, cassia oils, aniseed oils, bitter almond oils, clove oils, parsley seed oils, citrus oils, vanilla (extracts), or fruity flavoring compositions having tastes oriented towards, for example, apple, pear, peach, grape, strawberry, raspberry, cherry, or pineapple are preferably used.

In addition, suitable individual substances as part of the flavorants are those having a cooling refreshing effect in the throat or in the oral or nasal cavity. Non-limiting examples include menthol, menthone, menthone glycerin acetate, menthyl acetate, menthyl methyl ether, methone acetals, menthol carbonates, menthyl lactate, menthyl succinates (such as monomenthyl succinate sold under the tradename PHYSCOOL^{®}), substituted menthyl-3-carboxamides (for example menthyl-3-carboxylic acid-N-ethylamide), 2-isopropyl-N-2,3-trimethylbutanamide, substituted cyclohexane carboxamides, 3-menthoxypropane-1,2-diol, 2-hydroxyethyl menthyl carbonate, 2-hydroxypropyl menthyl carbonate, N-acetylglycine menthyl ester, isopulegol, hydroxycarboxylic acid menthyl esters (for example menthyl-3-hydroxybutyrate), 2-mercaptocyclodecanone, menthyl-2-pyrrolidin-5-onecarboxylate, 2,3-dihydroxy-p-menthane, 3,3,5-trimethylcyclohexanone glycerol ketal, 3-menthyl-3,6-di- and -tri-oxaalkanoates, 3-menthyl methoxyacetate, icilin, 1,8-cineol (eucalyptol), carvone, alpha-terpineol, thymol, methyl salicylate, 2'-hydroxypropiophenone, or a combination of two or more of the foregoing.

The oily liquid flavor composition may also comprise one or more hydrophobic sweeteners, with the use of solubilizing agents, if appropriate. In general, applicable sweeteners for the oily liquid flavor composition include sucralose, neotame, neohesperidin dihydrochalcone, or combinations thereof. Furthermore, other sweeteners, such as steviols, stevioside, rebaudioside A, glycyrrhizin, osladin, brazzein, miraculin, pentadin, phyllodulcin, dihydrochalcone, arylureas, trisubstituted guanidines, glycyrrhizin, superaspartam, suosan, sucralose (trichlorogalactosesucrose or TGS), alitame, monellin, as well as other natural or artificial sweeteners may also be used singly or in combinations. In an embodiment, the hydrophobic sweetener comprises sucralose, present in the oily liquid flavor composition in a sufficient quantity to provide about 0.1 to 2 wt%, preferably about 0.3 wt%, of sucralose in the dried gelatin bead.

If the oily liquid flavor composition is to be colored, suitable colorants include oil soluble colors, oil stable suspensions, or W/O emulsions. Nonlimiting examples of colors suitable for imparting color to the oily liquid flavor composition include lactoflavin (riboflavin), beta-carotene, riboflavin-5'-phosphate, alpha-carotene, gamma-carotene, cantaxanthin, erythrosine, curcumin, quinoline yellow, yellow orange S, tartrazine, bixin, norbixin (annatto, orlean), capsanthin, capsorubin, lycopene, beta-apo-8'-carotenal, beta-apo-8'-carotenic acid ethyl ester, xantophylls (flavoxanthin, lutein, cryptoxanthin, rubixanthin, violaxanthin, rodoxanthin), fast carmine (carminic acid, cochineal), azorubin, cochineal red A (Ponceau 4 R), beetroot red, betanin, anthocyanins, guaiazulene, amaranth, patent blue V, indigotine I (indigo-carmine), chlorophylls, copper compounds of chlorophylls, acid brilliant green BS (lissamine green), brilliant black BN, vegetable carbon, titanium dioxide, iron oxides and hydroxides, calcium carbonate, aluminum, silver, gold, pigment rubine BK (lithol rubine BK), methyl violet B, victoria blue R, victoria blue B, acilan brilliant blue FFR (brilliant wool blue FFR), naphthol green B, acilan fast green 10 G (alkali fast green 10 G), ceres yellow GRN, sudan blue II, ultramarine, phthalocyanine blue, phthalocayanine green, or fast acid violet R. Further naturally obtained colorants, such as those commercially available from Kancor Ingredients Ltd. (Kerala, India), e.g., anthocyanins, betanins, bixins, norbixins, carmines, carotenoids, chlorophyls, curcumins, spirulinas, etc., can be used for coloring purposes. The so-called aluminum lakes: FD & C Yellow 5 Lake, FD & C Blue 2 Lake, FD & C Blue 1 Lake, Tartrazine Lake, Quinoline Yellow Lake, FD & C Yellow 6 Lake, FD & C Red 40 Lake, Sunset Yellow Lake, Carmoisine Lake, Amaranth Lake, Ponceau 4R Lake, Erythrosyne Lake, Red 2G Lake, Allura Red Lake, Patent Blue V Lake, Indigo Carmine Lake, Brilliant Blue Lake, Brown HT Lake, Black PN Lake, Green S Lake, and mixtures thereof, may also be used.

Preferred antioxidants including substances which can reinforce an antioxidative effect are for example naturally occurring tocopherols and derivatives thereof (for example vitamin E-acetate), vitamin C and the salts or derivatives thereof (for example ascorbyl palmitate, Mg-ascorbyl phosphate, ascorbyl acetate), vitamin A and derivatives (vitamin A-palmitate), tocotrienols, flavonoids, alpha-hydroxy acids (for example citric acid, lactic acid, malic acid, tartaric acid) and the Na+, K+ and Ca+2 salts thereof, flavonoids, quercetin, phenolic benzylamines, propyl gallate, octyl gallate, dodecyl gallate, butylhydroxyanisol (BHA, E320), butyl hydroxytoluene (BHT, 2,6-di-tert-butyl-4-methyl-phenol, E321), lecithins, mono- and diglycerides of edible fatty acids esterified with citric acid, carotenoids, carotenes (for example α-carotene, β-carotene, lycopene) and derivatives thereof, phytic acid, lactoferrin, EDTA, EGTA), folic acid and derivatives thereof, ubiquinone and ubiquinol and derivatives thereof, ferulic acid and derivatives thereof, zinc and derivatives thereof (for example ZnO, ZnSO₄), selenium and derivatives thereof (for example selenium methionine), orthophosphates and Na+, K+, and Ca+2 salts of monophosphoric acid as well as ingredients isolated from plants, extracts or fractions thereof, for example, from tea, green tea, algae, grape seeds, wheatgerm, camomile, rosemary and oregano.

The oily liquid flavor composition may contain substances or substance mixtures active in nutritional physiology (nutraceuticals). Nutraceuticals in the meaning of the invention are substances or mixtures of substances that add a healthy benefit to the beads according to the invention. Examples of such substances are especially vitamins, minerals, trace elements, micronutrients, probiotics, and/or antioxidants. Non-limiting examples include panthenol, pantothenic acid, essential fatty acids, vitamin A and derivatives, carotenes, vitamin C (ascorbic acid), vitamin E (tocopherol) and derivatives, vitamins of the B and D series, such as vitamin B6 (nicotinamide), vitamin B12, vitamin D1, vitamin D3, vitamin F, folic acid, biotin, amino acids, oil soluble compounds of the elements magnesium, silicon, phosphorus, calcium, manganese, iron or copper, coenzyme Q10, unsaturated fatty acids, omega-3-fatty acids, polyunsaturated fatty acids, γ-linolenic acid, oleic acid, eicosapentaenoic acid, docosahexaenoic acid and derivatives thereof, bisabolene, chloramphenicol, caffeine, capsaicin, prostaglandins, thymol, camphor, y-oryzanol, salmon oil, mustard oil such as allyl isothiocyanate (AITC), oil soluble or oil miscible extracts, concretes or residues of plant and animal origin, or probiotics such as Bifidobacterium-containing compositions.

Antitussive actives can be added and include e.g. dextromethorphan, chlophedianol, carbetapentane, caramiphen, nosciapine, diphenylhydramine, codeine, hydrocodone, hydromorphone, fominoben and benzonatate. Oral anesthetic actives can be added and include e.g. phenol, lidocaine, dyclonine, benzocaine, menthol, salicyl alcohol and hexylresorcinol.

The oily liquid flavor composition may also comprise one or more weighting agents as used in aromatic emulsions, such as dammar gum, wood resins of the ester gum type, sucrose acetate isobutyrate (SAIB), or brominated vegetable oils. The function of these weighting agents is to adjust the density of the oily liquid flavor composition. Typical density of the oily liquid flavor composition is in a range between 0.8 to 1 g/ml, preferably 0.85 to 0.95 g/ml.

The oily liquid flavor composition may also include one or more captive agents, including but not limited to, Betahydrane^{™} (3-benzyl-tetrahydropyran); Antillone^{™} (9-decen-2-one); Noreenal^{™} ((±)-6,8-Dimethylnon-7-enal); and/or Pescagreen^{™} (2-(2,4,4-trimethyl-cyclopentyl)-acrylonitrile).

### EMULSION FORMATION

In accordance with embodiments of the present invention, the aqueous gellable mixture and the oily liquid flavor composition are mixed under high shear to provide a stable oil-in-water (O/W) emulsion comprising a plurality of oily liquid flavor composition droplets having an average diameter in a range from about 1 micron to about 20 microns dispersed therein. For example, the plurality of oily liquid flavor composition droplets may have an average diameter in a range of 1 micron to about 20 microns, 1 micron to about 15 microns, 1 micron to 10 microns, or 1 micron to 5 microns. Due to a lack of commercially-available particle size analyzers having heating capability, the average diameter of the oily liquid flavor composition droplets in the emulsion may be measured indirectly by scanning electron microscopy of the gelled matrix of the dried flavor filled bead. Mixing of the aqueous gellable mixture and the oily liquid flavor composition is performed at a temperature above the gelling temperature of the gellable mixture, in order to inhibit premature gel formation. Homogenizers or other high shear mixing apparatus are useful for this step. The resulting emulsion is stable for an extended period of time when maintained at a temperature above the gelling temperature of the gellable mixture.

### GELATIN BEAD FORMATION

In accordance with embodiments of the present invention, encapsulated flavor delivery system comprising spherical beads are formed using co-extrusion techniques. The general method includes preparing the gellable mixture, which will form the gelled matrix, and preparing an oily liquid flavor composition, which will form the plurality of oily liquid flavor composition droplets within the gelled matrix, and then under high shear mixing forming an oil-in-water (O/W) emulsion of the two. The emulsion if forced through a nozzle assembly thereby forming an emulsion stream.

In accordance with embodiments of the present invention, the discharge of the nozzle is directed into a cooled stream of non-aqueous fluid, which sufficiently lowers the temperature of the gellable mixture below its gelling temperature to induce gel formation. Suitable non-aqueous fluids include, but are not limited, to medium-chain fatty acid triglyceride (MCT), plant fatty acid (palm oil, sunflower oil, safflower oil, sesame oil, rapeseed oil, grape seed oil and a mixture thereof), liquid paraffin and a mixture thereof. In an embodiment, the non-aqueous fluid comprises medium chain triglycerides (MCT), such as MIGLYOL^{®} available from CREMER OLEO GMBH & CO, GERMANY.

In an embodiment, the emulsion composition is extruded through a nozzle immersed in the non-aqueous fluid, which has a temperature at least 10°C below a gelling temperature of the gellable mixture, to form wet spherical beads comprising a continuous gelled matrix of uncross-linked gelatin and filler. The continuous gelled matrix surrounds the plurality of droplets of the oily liquid flavor composition. The wet spherical beads may be isolated from the non-aqueous fluid and then dried to provide the encapsulated flavor delivery system comprising dried spherical beads having an average particle diameter in a range from about 400 microns to about 2000 microns, with a coefficient of variance of less than 15%.

In order to break the emulsion stream into spherical gellable particles having the desired particle size diameter, various vibrational, electrostatic, mechanical, or hydrodynamic methods may be utilized, of which the most commonly used method is vibrational. For example, expired US. Patent No. 4,251,195 to Suzuki et al. and assigned to Morishita Jintan Company, Ltd. describes the use of a ring or cylinder body that vibrates with a certain frequency along the lengthwise direction of the liquid stream, and thus imparts vibrational energy causing the formation of waves that ultimately break into spherical particles due to the interfacial tension of the fluids. Abandoned German Patent Application DE19617924A1 to Thorsten and assigned to Brace GmbH describes the induction of vibration excitation to the liquid being dripped before the nozzles or at least a short distance away from the nozzle device. The direct introduction of the vibration can happen in different ways: 1) mechanical vibration transmission of an elastic body or an elastic membrane in the nozzle assembly or in the supply line just before the nozzle assembly; 2) a vibrating plunger may be inserted into the nozzle assembly; or integrating a piezoelectric crystal or an ultrasonic probe into the nozzle assembly or in the supply line just before the nozzle. And PCT Application Publ. No. WO0213786 to Kim et al. and assigned to the Board of Trustees of the University of Illinois, describes implementing an acoustic-type vibrational wave to break an accelerated cylindrical jet of an extruded stream into droplets.

In an embodiment, the vibration energy may be applied to the emulsion. Alternatively, the vibrational energy may be applied to the nozzle. One or more of a variety of vibration methods, including but not limited to, acoustic vibration, vibrating nozzle, a piezoelectric vibrator, etc., breaks the emulsion stream into droplets having a size related to the vibration frequency.

In accordance with an aspect of the invention, the vibration frequency may be in a range from 50 Hz to 3500 Hz. For example, the vibration frequency may be 50 Hz, 75 Hz, 100 Hz, 200 Hz, 300 Hz, 400 Hz, 500 Hz, 600 Hz, 700 Hz, 800 Hz, 900 Hz, 1000 Hz, 1250 Hz, 1500 Hz, 1750 Hz, 2000 Hz, 2500 Hz, 3000 Hz, 3500 Hz, or in a range between any two of the foregoing. For example, the vibration frequency may be in a range from 200 to 2000 Hz.

Several other process parameters may be set or controlled, including the nozzle diameter, the feed rate of the emulsion stream, and the viscosity of the emulsion stream.

Thus, in accordance with an embodiment, the nozzle may have an inner diameter in a range from 100 microns to 1500 microns, such as 100 microns, 150 microns, 200 microns, 250 microns, 300 microns, 350 microns, 400 microns, 450 microns, 500 microns, 550 microns, 600 microns, 650 microns, 700 microns, 750 microns, 800 microns, 900 microns, 1000 microns, 1100 microns, 1200 microns, 1300 microns, 1400 microns, 1500 microns, 1600 microns, 1700 microns, 1800 microns, 1900 microns, 2000 microns, 2100 microns, 2200 microns, 2300 microns, 2400 microns, 2500 microns, 2600 microns, 2700 microns, 2800 microns, 2900 microns, 3000 microns, or in a range between any two of the foregoing.

In accordance with an embodiment, the feed rate of the emulsion composition through the nozzle may be in a range from 1 to 150 mL/min, such as 1 mL/min, 2 mL/min, 5 mL/min, 10 mL/min, 25 mL/min, 50 mL/min, 75 mL/min, 100 mL/min, 125 mL/min, 150 mL/min, 200 mL/min, 300 mL/min, 400 mL/min, 500 mL/min, or in a range between any two of the foregoing.

In accordance with embodiments of the present invention, the dynamic viscosity of the emulsion is in a range between from 5 to 350 mPa•sec, where the dynamic viscosity is measured at 60°C using MARSIII Haake Rheometer; cone 35mm/2°; shear rate 10s⁻¹. For example, the dynamic viscosity of the emulsion, measured at 70 °C, and shear of 10s⁻¹, may be 5 mPa•sec, 10 mPa•sec, 15 mPa•sec, 20 mPa•sec, 25 mPa•sec, 30 mPa•sec, 50 mPa•sec, 70 mPa•sec, 90 mPa•sec, 100 mPa•sec, 110 mPa•sec, 125 mPa•sec, 140 mPa•sec, 160 mPa•sec, 175 mPa•sec, 200 mPa•sec, 250 mPa•sec, 300 mPa•sec, 350 mPa•sec, or in a range between any two of the foregoing. In an embodiment, the dynamic viscosity of the emulsion, is in a range from 35 to 150 mPa•sec, or 20 to 80 mPa•sec, measured at 60 °C, and shear of 10s⁻¹.

The extrusion can be performed using extrusion equipment and processes similar to that described in expired U.S. Patent No. 5,882,680 by Takei assigned to Freund Industrial Co., Ltd or U.S. Patent No. 6,719,933 by Nakamura et al. assigned Chugai.

In order to avoid premature gelling of the emulsion and clogging of the extrusion nozzle, the emulsion is advantageously maintained at or above the gelling temperature of the gellable mixture. Accordingly, holding tank(s), transfer line(s), and/or the extrusion nozzle may be maintained at a desired temperature. In an embodiment, the holding tank and the transfer lines are heated to 65°C, which is above the gelling temperature of the gellable mixture. However, in accordance with embodiments of the present invention, the extrusion nozzle is immersed in the non-aqueous fluid, which has a temperature at least 10°C below a gelling temperature of the gellable mixture. For example, the non-aqueous fluid may be between 5°C and 40°C, such as 5°C, 10°C, 15°C, 20°C, 25°C, 30°C, 35°C, 40°C, or in a range between any two of the foregoing.

According to another embodiment of the invention, after the extrusion step the gelatin beads may be maintained in a chilled non-aqueous fluid bath in order to ensure further gelling of the gelled matrix. For example, the cold non-aqueous fluid bath may be a cold medium chain triglyceride (MCT) bath, which is maintained at a temperature at least 10 °C below the gelation temperature of the gellable mixture. For example, the bath temperature may be below 18 °C, such as about 2 °C to about 10 °C, or about 4 °C to about 6 °C, with a residence time to achieve the desired level of gelation.

If the chilled non-aqueous fluid bath is an oil, such as MCT and/or if the gelatin beads are extruded with a submerged extrusion nozzle into chilled oil, the gelatin beads may be centrifuged in order to remove the surplus oil. Additionally, or alternatively, the gelatin beads may be washed with organic solvent (such as acetone, ethyl acetate, ethanol, petroleum ether, etc.) to remove the surplus oil.

In an embodiment, the method further comprises drying the gelatin beads to a water content of 10 wt% or less, a water activity of 0.8 or less, or both. The gelatin beads may be dried in a current of air at controlled temperature and humidity. The relative humidity of the drying air may be in a range from 20% to 60%, preferably 30 to 50%; the temperature of the drying air may be in a range from 15 °C to 80 °C, preferably 35 °C to 55 °C. Furthermore, drying aids or dispersing agents may be utilized. Exemplary drying aids or dispersing agents include, but are not limited to over-dried starch, such as corn starch; or silica, such as Tixosil^{®} (Solvay USA Inc. Cranberry, NJ). For measuring the water content in weight percent, based on the entire weight of the dried capsules, a Karl Fisher titrator (Mettler model DL18) is suitable. Water activity value can be measured using a LabMaster-aw by Novasina AG (Lachen, Switzerland), at 25°C.

The gelatin beads manufactured in accordance with an embodiment of the invention are spherical or substantially spherical, are monodispersed in size (i.e., a coefficient of variance of 15% or less), and have an average dried particle diameter from 400 microns to about 2000 microns. For example, the average dried particle diameter of the gelatin beads may be 400 microns, 450 microns, 500 microns, 550 microns, 600 microns, 650 microns, 700 microns, 750 microns, 800 microns, 850 microns, 900 microns, 950 microns, 1000 microns, 1200 microns, 1400 microns, or 1500 microns, 1600 microns, 1800 microns, 2000 microns, or in a range between any two of the foregoing. In an embodiment, the average dried particle diameter is in a range between 500 microns to 1800 microns, 550 microns to 1600 microns, 600 microns to 1400 microns, or 800 microns to 1200 microns. In an embodiment, the coefficient of variance of average particle diameter of the dried gelatin beads is 15% or less, such as 14%, 13%, 12%, 11%, 10%, 5%, or less. Particle diameter measurements, including variance, may be measured using a Beckman Coulter LS 13 320 Particle Size Analyzer, optical model Fraunhofer.rf780f, using volume statistics (arithmetic) of the mean value.

The total weight of the dried gelatin beads of the invention depend on its diameter, gelled matrix content, flavor loading, and its final moisture content. According to an embodiment of the invention, the total weight of the dried gelatin bead is within the range of 0.03 to 5 mg, such as 0.3 to 4 mg, 0.4 to 3 mg, 0.5 to 2 mg. The oily liquid flavor loading within the dried gelatin beads may range from 30 wt% to 80 wt%, preferably 40 wt% to 75 wt%, more preferably 50 wt% to 70 wt%, based on the total weight of the dried gelatin beads.

According to a preferred embodiment, the dried gelatin beads according to the invention are characterized as having an initial modulus (g/mm²/%) of at least 7, preferably greater than 15. The initial modulus is measured on dried gelatin beads having a moisture content of 10% or less, and a water activity of 0.8 or less. The texture of the capsules may be characterized using a TA.XTplus texture analyzer from Stable Micro System Ltd. (Surrey, UK) in compression mode with a 5 Kg load cell; Probe: P0.5 - ½ diameter DELRIN^{®} cylinder; cylinder speed 0.5mm/sec; resolution of 0.01 Kg. The dried bead is positioned on the TA.XTplus device between the base and the probe. Vertical compressive force is then continuously applied onto one bead until the bead begins to break and simultaneously the built-in gauge records force (in kilograms (Kg) or newton (N)) and position (in millimeter (mm)).

In accordance with another embodiment of the present invention, a method for providing an extended release of an oily liquid flavor composition in a confectionary composition is provided. The method includes dispersing 0.1 wt% to 20 wt% of the gelatin flavor beads in the confectionary composition, wherein wt% is based on the entire weight of the confectionery composition. The dried gelatin beads having an average particle diameter in a range from 400 microns to 2000 microns with a coefficient of variance of less than 15%, wherein the dried gelatin beads comprises: a continuous gelled matrix comprising an uncross-linked gelatin and a plasticizer, said matrix surrounding a plurality of droplets comprising an oily liquid flavor composition containing a flavorant, wherein the continuous gelled matrix is substantially void of acid polysaccharide gelling agents. The plurality of flavor droplets have an average diameter in a range from about 1 micron to about 20 microns.

Confectionery products include chewable products comprising a sweetener selected from the group of monosaccharides, disaccharides, polysaccharides, polyol sweeteners, non-nutritive sweeteners, and combinations thereof. When oily liquid flavor compositions, which are encapsulated within the dried gelatin beads according to the present invention, are homogenously dispersed in the confectionary products, such as compressed chewing gum tablets or chewing gum mini-stick, the sensory experience for an encapsulated flavor in the confectionary composition is surprisingly enhanced and extended, relative to other encapsulated techniques. During mastication, the gelatin beads are broken thereby releasing their flavor contents. Applicants have discovered that the inventive monodispersed gelatin beads (having an average particle diameter and coefficient of variance within the ranges disclosed herein) provide an enhanced flavor-filled experience, both in intensity and longer lasting experience, relative to other flavor delivery systems. In an embodiment, the inventive monodispersed gelatin beads may be combined with free or other forms of encapsulated flavors to provide long lasting flavor to a confectionary product.

In an embodiment, the dried gelatin beads having an average particle diameter in a range from 800 microns to 1200 microns with a coefficient of variance of less than 10%, and a oily liquid flavor loading of at least 50 wt%.

In an embodiment, the gelatin beads include a hydrophilic sweetener homogenously dispersed throughout the continuous gelled matrix, or a hydrophobic sweetener homogenously dispersed throughout the oily liquid flavor composition. In an embodiment, the gelatin beads include a hydrophilic sweetener homogenously dispersed throughout the continuous gelled matrix, and a hydrophobic sweetener homogenously dispersed throughout the oily liquid flavor composition. Advantageously, a combination of sweeteners (hydrophilic sweetener in the gelled matrix and a hydrophobic sweetener in the oily liquid flavor) suppresses bitterness perception is certain flavors. In an embodiment, the hydrophilic sweetener comprises acesulfame potassium, present in the gellable mixture in a sufficient quantity to provide about 0.5 to 3 wt%, preferably about 1 wt%, of acesulfame potassium in the dried gelatin bead, and the hydrophobic sweetener comprises sucralose, present in the oily liquid flavor composition in a sufficient quantity to provide about 0.1 to 2 wt%, preferably about 0.3 wt%, of sucralose in the dried gelatin bead.

In an embodiment, the confectionary product comprises a chewing gum base, which can also contain any of a variety of traditional ingredients such as plasticizers or softeners such as lanolin, stearic acid, sodium stearate, potassium stearate, glyceryl triacetate, glycerine and the like and/or waxes, for example, natural waxes, petroleum waxes, such as polyethylene waxes, paraffin waxes and microcrystalline waxes, to obtain a variety of desirable textures and consistency properties. These individual additional materials are generally employed in amounts of up to about 30% by weight and preferably in amounts of from about 3% to about 20% by weight of the final chewing gum base composition. The chewing gum base composition may additionally include conventional additives such as emulsifiers such as lecithin and glyceryl monostearate; and additional fillers such as aluminum hydroxide, magnesium hydroxide, alumina, aluminum silicates, calcium carbonate, and talc and combinations thereof. These fillers may be used in the chewing gum base in various amounts. Preferably the amount of fillers when used will vary from about 4 to about 30% by weight of the final chewing gum base. The chewing gum embodiments of the present invention containing the flavor-filled, gelatin beads may further include a one or more flavor delivery systems selected from liquid, spray dry, spray dry granulation, seamless capsules, or other encapsulations techniques.

All the features described previously regarding the confectionary product also apply to the method of the invention.

Non-limiting examples of embodiments of the present invention, in accordance with the description and in comparison with embodiments outside the scope of the invention, are now disclosed below. These examples are merely for the purpose of illustration and are not to be regarded as limiting the scope of the invention or the manner in which it can be practiced. Other examples and/or applications will be appreciated by a person having ordinary skill in the art.

### EXAMPLES

Example 1 - A gellable mixture was prepared in a jacketed mixer by combining 23 g of sorbitol and 0.28 g of FDA Blue 1 with 1195 g of process water and warmed to 65 °C while gently stirring at 500 rpm, followed by dissolving 169 g of bovine gelatin (Bloom 280, 40 mesh) and degassing the formed solution of aqueous gellable mixture. The gellable mixture was then agitated under high shear (10,000 rpm) and 408 g of oily liquid peppermint flavor (density 0.935 g/ml) was slowly introduced to form the gelatin-flavor (O/W) emulsion, which was stirred for an additional 5 minutes and then maintained at 65 °C.

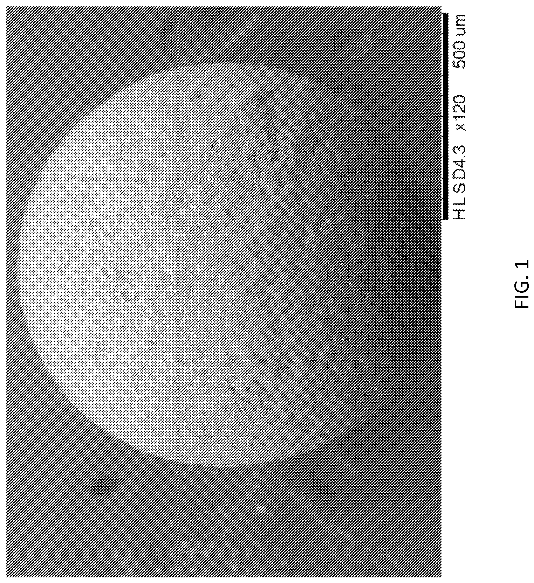

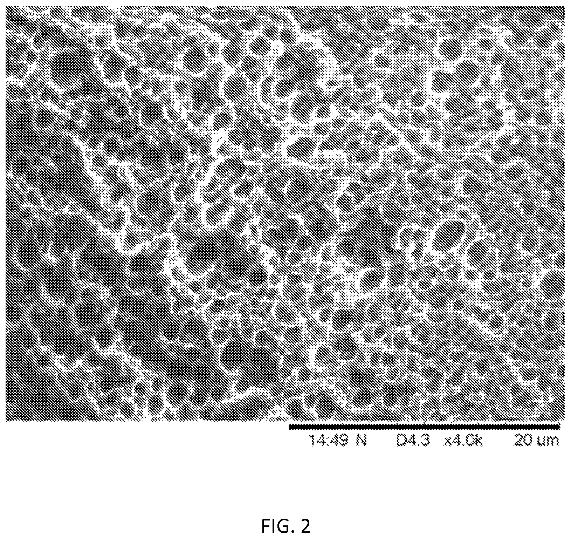

The gelatin-flavor emulsion was pushed through a needle (1.2 mm diameter) into an accelerated stream of MIGLYOL^{®} (at 15°C) at 20 ml/min to form wet beads having an average diameter of approximately 1.6 mm. The wet beads were chilled for about an hour at 7°C and then centrifuged at 3500 rpm to remove excess oil and combined with 3% of processing aid. The wet beads were dried in a fluid bed dryer between 35°C and 45°C until a final moisture content of less than 5 wt% was achieved. The dried gelatin beads were sieved on an oscillating-type sifter to remove the processing aid and provide 1.0 mm dried spherical beads. Referring now to FIG. 1, a typical bead was analyzed under a scanning electron microscope (SEM) at 120x magnification and show a spherical shape with a generally smooth outer surface with minor variations, whereas the SEM image shown in FIG. 2 of a broken bead showing the internal structure (cross-section) of the continuous gelled matrix surrounding the plurality of oily flavor droplets.

Comparative Example 1. Seamless core-shell capsules were prepared by coextruding the gellable mixture and the oily liquid peppermint flavor of Example 1 (partially diluted with MCT) to provide 1.1 mm traditional capsules.

Comparative Example 2: Spray dried flavor composition was prepared using a modified starch, maltodextrin, and the oily liquid peppermint flavor (undiluted) utilized in Example 1. The spray dried flavor composition has an average particle size diameter of approximately 60 microns with approximately 20 wt% flavor loading.

Examples 2a - 2d - Processing parameters substantially similar to Example 1 were utilized to make inventive Examples 2a-2d, except using a spearmint oily liquid flavor. Four variations were prepared: No artificial sweetener added to the gellable mixture or spearmint flavor (example 2a); adding a hydrophobic artificial sweetener (sufficient amount to provide 0.33 wt% sucralose in dried bead) to the spearmint flavor (example 2b); adding a hydrophilic sweetener (sufficient amount to provide 1.01 wt% AceK in the dried bead) to the gellable matrix (example 2d); and adding artificial sweeteners to both the spearmint flavor (0.5 wt% sucralose) and the gellable mixture (1.01 wt% AceK) (example 2c).

A summary of various physical and characteristic details relating to encapsulated flavor Examples 1 and 2a-2d, and Comparative Examples 1 and 2 are detailed in Table 1 (below).

**TABLE 1: Encapsulated Flavors used in Sugar-free Chewing Gum Testing.**

| **Example** | **Gel Agent** | **Filler** | **Avg Diam (mm)** | **Variance (%)** | **Relative mass for equiv. flavor active** | **% H₂O** | **Sweetener added** |
|---|---|---|---|---|---|---|---|
| 1 | Gelatin | Sorbitol | 1.0 | <15 | 1 | <3 | None |
| Comp 1 | Gelatin | Sorbitol | 1.1 | <15 | 1.3 | <3 | None |
| Comp 2 | none | Mod.Starch/ Maltodextrin | 0.06 | ~ | 3.3 | <3 | None |
| 2a | Gelatin | Sorbitol | 1.01 | <15 | 1 | <5 | None |
| 2b | Gelatin | sorbitol | 1.0 | <15 | 1 | <5 | 0.33 wt% Sucralose in flavor |
| 2c | Gelatin | Sorbitol | 1.05 | <15 | 1 | <5 | 0.33 wt% Sucralose in flavor & 1.01 wt% AceK in matrix |
| 2d | Gelatin | Sorbitol | 1.06 | <15 | 1 | <5 | 1.01 wt% AceK in matrix |

Sugar-free chewing gum samples were prepared in accordance with the formulation shown in Table 2, by mixing the various ingredients 1-7 at 80°C to 85°C until homogenous, and then adding encapsulated flavors (ingredients 8a-8g), followed by artificial sweeteners (ingredients 9-11) and mixing for a few additional minutes prior to cooling, and then forming sheets of a desired thickness and sticks of desired dimensions. Chewing gum samples were aged approximately two weeks prior to sensory testing.

**TABLE 2: Sugar Free Chewing Gum Formulation.**

| | | **Gum A** | **Gum B** | **Gum C** | **Gum D** | **Gum E** | **Gum F** | **Gum G** |
|---|---|---|---|---|---|---|---|---|
| | **Ingredients** | **wt%** | **wt%** | **wt%** | **wt%** | **wt%** | **wt%** | **wt%** |
| 1 | P60W Crystalline Sorbitol | 48.65 | 48.50 | 47.50 | 46.21 | 46.21 | 46.21 | 46.21 |
| 2 | Gum Base | 26.00 | 26.00 | 26.00 | 32.00 | 32.00 | 32.00 | 32.00 |
| 3 | Liquid Sorbitol | 3.00 | 3.00 | 3.00 | - | - | - | - |
| 4 | Glycerine 99% | 7.00 | 7.00 | 7.00 | 7.00 | 7.00 | 7.00 | 7.00 |
| 5 | Mannitol Powder | 4.00 | 4.00 | 4.00 | 5.00 | 5.00 | 5.00 | 5.00 |
| 6 | Maltitol Syrup | 10.00 | 10.00 | 10.00 | 8.50 | 8.50 | 8.50 | 8.50 |
| 7 | Soy Lecithin | 0.35 | 0.35 | 0.35 | - | - | - | - |
| 8a | Flavor Ex 1 | 0.5 | | | | | | |
| 8b | Flavor Comp Ex 1 | | 0.65 | | | | | |
| 8c | Flavor Comp Ex 2 | | | 1.65 | | | | |
| 8d | Flavor Ex 2a | | | | 1.00 | | | |
| 8e | Flavor Ex 2b | | | | | 1.00 | | |
| 8f | Flavor Ex 2c | | | | | | 1.00 | |
| 8g | Flavor Ex 2d | | | | | | | 1.00 |
| 9 | Aspartame | 0.35 | 0.35 | 0.35 | 0.15 | 0.15 | 0.15 | 0.15 |
| 10 | Acesulfame K | 0.15 | 0.15 | 0.15 | 0.10 | 0.10 | 0.10 | 0.10 |
| 11 | Sucralose | - | - | - | 0.04 | 0.04 | 0.04 | 0.04 |
| | **Total** | 100.0 0 | 100.0 0 | 100.0 0 | 100.0 0 | 100.0 0 | 100.0 0 | 100.00 |

Sugar-free chewing gum samples A-C were evaluated in duplicate by a trained panel of 12 panelists over a two (2) day evaluation period. Panelists aligned their rate of chew with a metronome that was programmed to sound every second. The rate of chew was 1 chew per second (60 chews per minute). Panelists rated the Overall Flavor Intensity on an 11-point scale (0 = None and 10 = High) using paper ballots. Panelists scored the samples every 30 seconds for the first 2 minutes and then every minute throughout the remaining 6-minute chewing period. Mean intensity values were graphed at each time interval. Panelists cleansed their palate with unflavored, carbonated water and milk chocolate during timed breaks between samples.

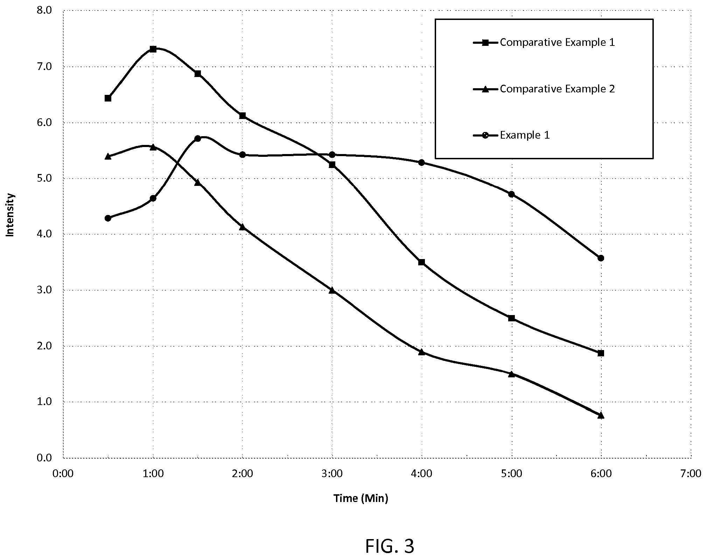

As shown in FIG. 3, panelist found that, for the same overall flavor actives loading, the inventive dried flavor filled beads provided higher flavor intensity experience a) after approximately 1.5 minutes versus a traditional spray dried encapsulated delivery system; and b) after 3 minutes versus a traditional core-shell encapsulated delivery system.

Sugar-free spearmint-flavored chewing gum samples D-G containing inventive gelatin beads 2a-2d were evaluated by a trained panel of 12 panelists to assess bitterness. Panelists aligned their rate of chew to approximately 1 chew per second (60 chews per minute). Panelists recorded the presence or absence of bitterness of each sample using paper ballots, recorded at 5 seconds, 15 seconds, 30 seconds, 45 seconds, and 1 minute, then at 30 seconds intervals for the next two minutes, and then every minute throughout the remaining 8 minute chewing period. The percentage of panelists perceiving bitterness were graphed at each time interval. Panelists cleansed their palate with unflavored, carbonated water and milk chocolate during timed breaks between samples.

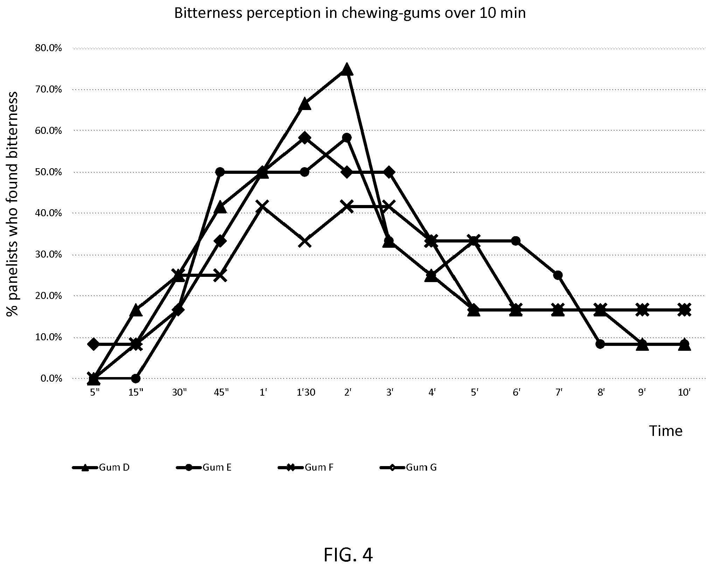

As shown in FIG. 4, panelist found that Gum F containing the inventive dried spearmint-flavor filled gelatin bead (Ex. 2c) comprising a combination of a hydrophilic sweetener (AceK) in the gelled matrix and a hydrophobic sweetener (sucralose) in the oily flavor droplets suppressed the bitterness perception (30 seconds to 3 minutes) in a sugar-free chewing gum application versus no sweetener in the bead (Gum D; Ex. 2a) or only putting sweetener in one of the gelled matrix or oily flavor (Gums E or G; Ex. 2b or Ex. 2d).

While the invention has been illustrated by the description of one or more embodiments thereof, and while the embodiments have been described in considerable detail, they are not intended to restrict or in any way limit the scope of the appended claims to such detail. Additional advantages and modifications will readily appear to those skilled in the art. The invention in its broader aspects is therefore not limited to the specific details, representative product and/or method and examples shown and described. The various features of exemplary embodiments described herein may be used in any combination. Accordingly, departures may be made from such details without departing from the scope of the general inventive concept.

## Claims

1. An encapsulated flavor delivery system, comprising:
a plurality of dried spherical beads, each in the form of a continuous gelled matrix comprising an uncross-linked gelatin and a filler, said matrix surrounding a plurality of droplets comprising an oily liquid flavor composition containing a flavorant, wherein the continuous gelled matrix is substantially void of acid polysaccharide gelling agents, said continuous gelled matrix comprising less than 1 wt% of acid polysaccharide gelling agents, based on the entire weight of the continuous gelled matrix,
wherein the plurality of droplets has an average diameter in a range from about 1 micron to about 20 microns, and
wherein the plurality of dried spherical beads has an average particle diameter in a range from about 400 microns to about 2000 microns, with a coefficient of variance less than 15%.

2. The encapsulated flavor delivery system according to claim 1, further comprising:
i) a hydrophilic sweetener homogenously dispersed throughout the continuous gelled matrix,
ii) a hydrophobic sweetener homogenously dispersed throughout the oily liquid flavor composition, or
iii) a hydrophilic sweetener homogenously dispersed throughout the continuous gelled matrix and a hydrophobic sweetener homogenously dispersed throughout the oily liquid flavor composition.

3. The encapsulated flavor delivery system according to claim 1 or 2, wherein the oily liquid flavor composition further comprises a medium chain triglyceride having a melting point of about 30°C or less.

4. The encapsulated flavor delivery system according to any one of claims 1 to 3, wherein the filler is selected from the group consisting of starch derivatives, cellulose derivatives, a polyvinyl alcohol, polyols with non-plasticizing properties, polyols with plasticizing properties, and combinations thereof.

5. The encapsulated flavor delivery system according to any one of claims 1 to 4, wherein the continuous gelled matrix comprising the uncross-linked gelatin and the filler is obtained from a gelatin having a Bloom value between 150 and 300.

6. The encapsulated flavor delivery system according to any one of claims 1 to 5, wherein the mass of the continuous gelled matrix is 20 wt% to 70 wt%, and wherein the mass of the oily liquid flavor composition is 30 wt% to 80 wt%, wherein wt% is based on the total mass of the spherical bead, excluding any water mass.

7. A method for making an encapsulated flavor delivery system, the method comprising:
a. forming an emulsion composition comprising a plurality of droplets comprising an oily liquid flavor composition containing a flavorant within a gellable mixture comprising an aqueous solution of gelatin and a filler, wherein the plurality of droplets has an average diameter in a range from about 1 micron to about 20 microns;
b. extruding the emulsion composition through a nozzle immersed in a non-aqueous fluid at a temperature at least 10°C below a gelling temperature of the gellable mixture to form wet spherical beads comprising a continuous gelled matrix comprising uncross-linked gelatin and the filler, said matrix surrounding the plurality of droplets of the oily liquid flavor composition;
c. isolating the wet spherical beads from the non-aqueous fluid; and
d. drying the wet spherical beads to form the encapsulated flavor delivery system comprising a plurality of dried spherical beads having an average particle diameter in a range from about 400 microns to about 2000 microns, with a coefficient of variance less than 15%; wherein the gellable mixture is substantially void of acid polysaccharide gelling agents such that the continuous gelled matrix comprises less than 1 wt% of acid polysaccharide gelling agents, based on the entire weight of the continuous gelled matrix.

8. The method according to claim 7, wherein the encapsulated flavor delivery system further comprises:
i) a hydrophilic sweetener homogenously dispersed throughout the continuous gelled matrix,
ii) a hydrophobic sweetener homogenously dispersed throughout the oily liquid flavor composition, or
iii) a hydrophilic sweetener homogenously dispersed throughout the continuous gelled matrix and a hydrophobic sweetener homogenously dispersed throughout the oily liquid flavor composition.

9. The method according to claim 7 or 8, wherein the oily liquid flavor composition further comprising a medium chain triglyceride having a melting point of about 30°C or less.

10. The method according to any one of claims 7 to 9, wherein the gelatin has a Bloom value between 150 and 300.

11. The method according to any one of claims 7 to 10, wherein the mass of the continuous gelled matrix is 20 wt% to 70 wt%, and wherein the mass of the oily liquid flavor composition is 30 wt% to 80 wt%, wherein wt% is based on the total mass of the dried spherical bead, excluding any water mass.

12. A confectionary product, comprising the encapsulated flavor delivery system of any one of claims 1 to 6.

13. The confectionary product of claim 12, comprising a chewing gum matrix, wherein the continuous gelled matrix allows to release the flavorant while chewing for a controlled period of time and thereafter to release substantially all of its original flavor notes at desired flavor levels over a sustained period of time.

14. The confectionary product of claim 12 or 13, wherein the encapsulated flavor delivery system is prepared, or susceptible of being obtained, by the method of any one of claims 7 to 11.

15. Use of the encapsulated flavor delivery system according to any one of claims 1 to 6, or susceptible of being obtained by the method of any one of claims 7 to 11, for providing a sustained release of the original flavor notes of a confectionary product comprising the encapsulated flavor delivery system.

## Patentansprüche

1. Ein verkapseltes Geschmacksabgabesystem, das Folgendes beinhaltet:
eine Vielzahl von getrockneten sphärischen Kügelchen, jeweils in Form einer kontinuierlichen gelierten Matrix, die eine unvernetzte Gelatine und einen Füllstoff beinhaltet, wobei die Matrix eine Vielzahl von Tröpfchen umgibt, die eine ölige flüssige Geschmackszusammensetzung, welche einen Geschmacksstoff enthält, beinhalten,
wobei die kontinuierliche gelierte Matrix im Wesentlichen frei von sauren Polysaccharidgeliermitteln ist, wobei die kontinuierliche gelierte Matrix bezogen auf das ganze Gewicht der kontinuierlichen gelierten Matrix zu weniger als 1 Gew.-% saure Polysaccharidgeliermittel beinhaltet,
wobei die Vielzahl von Tröpfchen einen mittleren Durchmesser in einem Bereich von etwa 1 Mikrometer bis etwa 20 Mikrometer aufweist, und
wobei die Vielzahl von getrockneten sphärischen Kügelchen einen mittleren Partikeldurchmesser in einem Bereich von etwa 400 Mikrometern bis etwa 2000 Mikrometer mit einem Streuungskoeffizienten von weniger als 15 % aufweist.

2. Verkapseltes Geschmacksabgabesystem gemäß Anspruch 1, das ferner Folgendes beinhaltet:
i) ein hydrophiles Süßungsmittel, das homogen in der gesamten kontinuierlichen gelierten Matrix verteilt ist,
ii) ein hydrophobes Süßungsmittel, das homogen in der gesamten öligen flüssigen Geschmackszusammensetzung verteilt ist, oder
iii) ein hydrophiles Süßungsmittel, das homogen in der gesamten kontinuierlichen gelierten Matrix verteilt ist, und ein hydrophobes Süßungsmittel, das homogen in der gesamten öligen flüssigen Geschmackszusammensetzung verteilt ist.

3. Verkapseltes Geschmacksabgabesystem gemäß Anspruch 1 oder 2, wobei die ölige flüssige Geschmackszusammensetzung ferner ein mittelkettiges Triglycerid mit einem Schmelzpunkt von etwa 30 °C oder weniger beinhaltet.

4. Verkapseltes Geschmacksabgabesystem gemäß einem der Ansprüche 1 bis 3, wobei der Füllstoff aus der Gruppe ausgewählt ist, die aus Stärkederivaten, Cellulosederivaten, einem Polyvinylalkohol, Polyolen mit nichtweichmachenden Eigenschaften, Polyolen mit weichmachenden Eigenschaften und Kombinationen davon besteht.

5. Verkapseltes Geschmacksabgabesystem gemäß einem der Ansprüche 1 bis 4, wobei die kontinuierliche gelierte Matrix, die die unvernetzte Gelatine und den Füllstoff beinhaltet, aus einer Gelatine mit einem Bloomwert von zwischen 150 und 300 erhalten wird.

6. Verkapseltes Geschmacksabgabesystem gemäß einem der Ansprüche 1 bis 5, wobei die Masse der kontinuierlichen gelierten Matrix 20 Gew.-% bis 70 Gew.-% ausmacht und wobei die Masse der öligen flüssigen Geschmackszusammensetzung 30 Gew.-% bis 80 Gew.-% ausmacht, wobei Gew.-% auf die Gesamtmasse des sphärischen Kügelchens, ausgenommen jegliche Wassermasse, bezogen ist.

7. Ein Verfahren zum Produzieren eines verkapselten Geschmacksabgabesystems, wobei das Verfahren Folgendes beinhaltet:
a. Bilden einer Emulsionszusammensetzung, die innerhalb einer gelierbaren Mischung, welche eine wässrige Lösung von Gelatine und einem Füllstoff beinhaltet, eine Vielzahl von Tröpfchen beinhaltet, welche eine ölige flüssige Geschmackszusammensetzung beinhalten, die einen Geschmacksstoff enthält, wobei die Vielzahl von Tröpfchen einen mittleren Durchmesser in einem Bereich von etwa 1 Mikrometer bis etwa 20 Mikrometer aufweist;
b. Extrudieren der Emulsionszusammensetzung durch eine in ein nichtwässriges Fluid eingetauchte Düse bei einer Temperatur von mindestens 10 °C unterhalb einer Geliertemperatur der gelierbaren Mischung, um nasse sphärische Kügelchen zu bilden, die eine kontinuierliche gelierte Matrix beinhalten, welche unvernetzte Gelatine und den Füllstoff beinhaltet, wobei die Matrix die Vielzahl von Tröpfchen der öligen flüssigen Geschmackszusammensetzung umgibt;
c. Isolieren der nassen sphärischen Kügelchen aus dem nichtwässrigen Fluid; und
d. Trocknen der nassen sphärischen Kügelchen, um das verkapselte Geschmacksabgabesystem zu bilden, das eine Vielzahl von getrockneten sphärischen Kügelchen mit einem mittleren Partikeldurchmesser in einem Bereich von etwa 400 Mikrometern bis etwa 2000 Mikrometer mit einem Streuungskoeffizienten von weniger als 15 % beinhaltet;
wobei die gelierbare Mischung im Wesentlichen frei von sauren Polysaccharidgeliermitteln ist, sodass die kontinuierliche gelierte Matrix bezogen auf das ganze Gewicht der kontinuierlichen gelierten Matrix zu weniger als 1 Gew.-% saure Polysaccharidgeliermittel beinhaltet.

8. Verfahren gemäß Anspruch 7, wobei das verkapselte Geschmacksabgabesystem ferner Folgendes beinhaltet:
i) ein hydrophiles Süßungsmittel, das homogen in der gesamten kontinuierlichen gelierten Matrix verteilt ist,
ii) ein hydrophobes Süßungsmittel, das homogen in der gesamten öligen flüssigen Geschmackszusammensetzung verteilt ist, oder
iii) ein hydrophiles Süßungsmittel, das homogen in der gesamten kontinuierlichen gelierten Matrix verteilt ist, und ein hydrophobes Süßungsmittel, das homogen in der gesamten öligen flüssigen Geschmackszusammensetzung verteilt ist.

9. Verfahren gemäß Anspruch 7 oder 8, wobei die ölige flüssige Geschmackszusammensetzung ferner ein mittelkettiges Triglycerid mit einem Schmelzpunkt von etwa 30 °C oder weniger beinhaltet.

10. Verfahren gemäß einem der Ansprüche 7 bis 9, wobei die Gelatine einen Bloomwert von zwischen 150 und 300 aufweist.

11. Verfahren gemäß einem der Ansprüche 7 bis 10, wobei die Masse der kontinuierlichen gelierten Matrix 20 Gew.-% bis 70 Gew.-% ausmacht und wobei die Masse der öligen flüssigen Geschmackszusammensetzung 30 Gew.-% bis 80 Gew.-% ausmacht, wobei Gew.-% auf die Gesamtmasse der sphärischen Kügelchen, ausgenommen jegliche Wassermasse, bezogen ist.

12. Ein Süßwarenprodukt, das das verkapselte Geschmacksabgabesystem gemäß einem der Ansprüche 1 bis 6 beinhaltet.

13. Süßwarenprodukt gemäß Anspruch 12, das eine Kaugummimatrix beinhaltet, wobei die kontinuierliche gelierte Matrix ermöglicht, dass der Geschmacksstoff während des Kauens über einen kontrollierten Zeitraum freigesetzt wird und danach im Wesentlichen alle seine ursprünglichen Geschmacksnoten in gewünschten Geschmacksstufen über einen anhaltenden Zeitraum freigesetzt werden.

14. Süßwarenprodukt gemäß Anspruch 12 oder 13, wobei das verkapselte Geschmacksabgabesystem durch das Verfahren gemäß einem der Ansprüche 7 bis 11 hergestellt wird oder dadurch erhältlich ist.

15. Verwendung des verkapselten Geschmacksabgabesystems gemäß einem der Ansprüche 1 bis 6 oder erhältlich durch das Verfahren gemäß einem der Ansprüche 7 bis 11 zum Bereitstellen einer anhaltenden Freisetzung der ursprünglichen Geschmacksnoten eines Süßwarenprodukts, das das verkapselte Geschmacksabgabesystem beinhaltet.

## Revendications

1. Un système de distribution d'arôme encapsulé, comprenant :
une pluralité de billes sphériques séchées, chacune sous la forme d'une matrice gélifiée continue comprenant une gélatine non réticulée et une charge, ladite matrice entourant une pluralité de gouttelettes comprenant une composition d'arôme liquide huileux contenant un agent aromatisant, où la matrice gélifiée continue est substantiellement dépourvue d'agents gélifiants à base de polysaccharide acide, ladite matrice gélifiée continue comprenant moins de 1 % en poids d'agents gélifiants à base de polysaccharide acide, rapporté à l'ensemble du poids de la matrice gélifiée continue,
dans lequel la pluralité de gouttelettes a un diamètre moyen compris dans une fourchette allant d'environ 1 micron à environ 20 microns, et
dans lequel la pluralité de billes sphériques séchées a un diamètre de particules moyen compris dans une fourchette allant d'environ 400 microns à environ 2 000 microns,
avec un coefficient de variance inférieur à 15 %.

2. Le système de distribution d'arôme encapsulé selon la revendication 1, comprenant en sus :
i) un édulcorant hydrophile dispersé de manière homogène dans toute la matrice gélifiée continue,
ii) un édulcorant hydrophobe dispersé de manière homogène dans toute la composition d'arôme liquide huileux, ou
iii) un édulcorant hydrophile dispersé de manière homogène dans toute la matrice gélifiée continue et un édulcorant hydrophobe dispersé de manière homogène dans toute la composition d'arôme liquide huileux.

3. Le système de distribution d'arôme encapsulé selon la revendication 1 ou la revendication 2, dans lequel la composition d'arôme liquide huileux comprend en sus un triglycéride à chaîne moyenne ayant un point de fusion d'environ 30 °C ou moins.

4. Le système de distribution d'arôme encapsulé selon l'une quelconque des revendications 1 à 3, dans lequel la charge est sélectionnée dans le groupe constitué de dérivés de l'amidon, de dérivés de la cellulose, d'un alcool polyvinylique, de polyols à propriétés non plastifiantes, de polyols à propriétés plastifiantes, et de combinaisons de ceux-ci.

5. Le système de distribution d'arôme encapsulé selon l'une quelconque des revendications 1 à 4, dans lequel la matrice gélifiée continue comprenant la gélatine non réticulée et la charge est obtenue à partir d'une gélatine ayant une valeur Bloom comprise entre 150 et 300.

6. Le système de distribution d'arôme encapsulé selon l'une quelconque des revendications 1 à 5, dans lequel la masse de la matrice gélifiée continue est de 20 % en poids à 70 % en poids, et dans lequel la masse de la composition d'arôme liquide huileux est de 30 % en poids à 80 % en poids, le % en poids étant rapporté à la masse totale de la bille sphérique, à l'exclusion de toute masse d'eau.

7. Un procédé pour la réalisation d'un système de distribution d'arôme encapsulé, le procédé comprenant le fait :
a. de former une composition d'émulsion comprenant une pluralité de gouttelettes comprenant une composition d'arôme liquide huileux contenant un agent aromatisant à l'intérieur d'un mélange gélifiable comprenant une solution aqueuse de gélatine et une charge, où la pluralité de gouttelettes a un diamètre moyen compris dans une fourchette allant d'environ 1 micron à environ 20 microns ;
b. d'extruder la composition d'émulsion à travers une buse immergée dans un fluide non aqueux à une température au moins 10 °C en dessous d'une température de gélification du mélange gélifiable afin de former des billes sphériques humides comprenant une matrice gélifiée continue comprenant de la gélatine non réticulée et la charge, ladite matrice entourant la pluralité de gouttelettes de la composition d'arôme liquide huileux ;
c. d'isoler les billes sphériques humides du fluide non aqueux ; et
d. de sécher les billes sphériques humides afin de former le système de distribution d'arôme encapsulé comprenant une pluralité de billes sphériques séchées ayant un diamètre de particules moyen compris dans une fourchette allant d'environ 400 microns à environ 2 000 microns, avec un coefficient de variance inférieur à 15 % ;
dans lequel le mélange gélifiable est substantiellement dépourvu d'agents gélifiants à base de polysaccharide acide de telle sorte que la matrice gélifiée continue comprend moins de 1 % en poids d'agents gélifiants à base de polysaccharide acide, rapporté à l'ensemble du poids de la matrice gélifiée continue.

8. Le procédé selon la revendication 7, dans lequel le système de distribution d'arôme encapsulé comprend en sus :
i) un édulcorant hydrophile dispersé de manière homogène dans toute la matrice gélifiée continue,
ii) un édulcorant hydrophobe dispersé de manière homogène dans toute la composition d'arôme liquide huileux, ou
iii) un édulcorant hydrophile dispersé de manière homogène dans toute la matrice gélifiée continue et un édulcorant hydrophobe dispersé de manière homogène dans toute la composition d'arôme liquide huileux.

9. Le procédé selon la revendication 7 ou la revendication 8, dans lequel la composition d'arôme liquide huileux comprend en sus un triglycéride à chaîne moyenne ayant un point de fusion d'environ 30 °C ou moins.

10. Le procédé selon l'une quelconque des revendications 7 à 9, dans lequel la gélatine a une valeur Bloom comprise entre 150 et 300.

11. Le procédé selon l'une quelconque des revendications 7 à 10, dans lequel la masse de la matrice gélifiée continue est de 20 % en poids à 70 % en poids, et dans lequel la masse de la composition d'arôme liquide huileux est de 30 % en poids à 80 % en poids, le % en poids étant rapporté à la masse totale de la bille sphérique séchée, à l'exclusion de toute masse d'eau.

12. Un produit de confiserie, comprenant le système de distribution d'arôme encapsulé de l'une quelconque des revendications 1 à 6.

13. Le produit de confiserie de la revendication 12, comprenant une matrice de chewing-gum, dans lequel la matrice gélifiée continue permet de libérer l'agent aromatisant lors de la mastication pendant un laps de temps contrôlé et par la suite de libérer substantiellement la globalité de ses notes d'arôme d'origine à des niveaux d'arôme souhaités sur un laps de temps prolongé.

14. Le produit de confiserie de la revendication 12 ou de la revendication 13, dans lequel le système de distribution d'arôme encapsulé est préparé, ou susceptible d'être obtenu, par le procédé de l'une quelconque des revendications 7 à 11.

15. Utilisation du système de distribution d'arôme encapsulé selon l'une quelconque des revendications 1 à 6, ou susceptible d'être obtenu par le procédé de l'une quelconque des revendications 7 à 11, pour fournir une libération prolongée des notes d'arôme d'origine d'un produit de confiserie comprenant le système de distribution d'arôme encapsulé.
